# EUROPEAN PATENT APPLICATION

(11) **EP 4 461 248 A1**
(43) Date of publication of application: **13.11.2024**
(21) Application number: 24175004.1
(22) Date of filing: 09.05.2024
(51) Int. Cl.: A61B 18/14, A61B 5/00

(54) **MULTI-PIECE BASKET CATHETER COMPRISING HINGES**

(30) Priority: 10.05.2023 US 202363501183 P; 09.04.2024 US 202418630968
(71) Applicant: Biosense Webster (Israel) Ltd., 2066717 Yokneam (IL)
(72) Inventor: XU, Guo, Irvine, 92618 (US); JIMENEZ, Jose, Irvine, 92618 (US); RORICK, Kevin, Irvine, 92618 (US)
(74) Representative: Carpmaels & Ransford LLP

(57) **Abstract**

The disclosed technology includes a medical probe comprising a tubular shaft including a proximal end and a distal end and an expandable basket assembly coupled to the distal end of the tubular shaft. The basket assembly can comprise a first plurality of spines coupled to the distal end of the tubular shaft and a second plurality of spines, each spine of the second plurality of spines rotatably coupled to a respective spine of the first plurality of spines. The basket assembly can include a plurality of electrodes attached to at least the second plurality of spines and a push rod connected to a distal end of the second plurality of spines. The push rod can be configured to slide longitudinally between a proximal position and a distal position to transition the expandable basket assembly between an expanded configuration and a collapsed configuration.

## Description

### CROSS-REFERENCE TO RELATED APPLICATION

This application claims, under 35 U.S.C. § 119(e), priority to and the benefit of U.S. Provisional Patent Application No. 63/501,183, filed May 10, 2023 (Attorney Docket No.: BIO6834USPSP1 - 253757.000373), the entire contents of which are incorporated herein by reference.

### FIELD

The present invention relates generally to medical devices, and in particular basket catheters configured to transition between a collapsed configuration and an expanded configuration.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another. Medical probes may utilize radiofrequency (RF) electrical energy to heat tissue. Some ablation approaches use irreversible electroporation (IRE) to ablate cardiac tissue using nonthermal ablation methods.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Furthermore, multiple linear spines are generally assembled together by attaching ends of the linear spines to a tubular shaft (e.g., a pusher tube) to form a spherical basket. Some basket catheters can take up a lot of space and be difficult to insert through an insertion catheter. Furthermore, due to the configuration of the basket, it is possible for the spines to break when being inserted or retracted through the sheath or manipulated. Accordingly, there is a need in the art for alternative designs of basket catheters that help to reduce the likelihood that a spine will break. This and other issues can be addressed by the technology disclosed herein.

### SUMMARY

The disclosed technology includes a medical probe comprising a tubular shaft including a proximal end and a distal end and an expandable basket assembly coupled to the distal end of the tubular shaft. The basket assembly can comprise a first plurality of spines coupled to the distal end of the tubular shaft and a second plurality of spines, each spine of the second plurality of spines rotatably coupled to a respective spine of the first plurality of spines. The basket assembly can include a plurality of electrodes attached to at least the second plurality of spines and a push rod connected to a distal end of the second plurality of spines. The push rod can be configured to slide longitudinally between a proximal position and a distal position to transition the expandable basket assembly between an expanded configuration and a collapsed configuration.

The disclosed technology includes a medical probe comprising a tubular shaft including a proximal end and a distal end. The tubular shaft can extend along a longitudinal axis. The medical probe can include an expandable basket assembly coupled to the distal end of the tubular shaft. The basket assembly can comprise a first plurality of spines coupled to the distal end of the tubular shaft and a second plurality of spines. Each spine of the second plurality of spines can be rotatably coupled to a respective spine of the first plurality of spines. The second plurality of spines can be biased to bow distally with respect to the first plurality of spines to cause the expandable basket assembly to transition to an expanded configuration.

The disclosed technology can include method of constructing a medical probe. The method can comprise attaching a first plurality of spines to a tubular shaft. The tubular shaft can extend along a longitudinal axis. The method can further include attaching a second plurality of spines to a distal end of the first plurality of spines. The second plurality of spines can be rotatably attachable to the first plurality of spines. The first plurality of spines and the second plurality of spines can form an expandable basket assembly. The method can further include attaching a distal end of the second plurality of spines to a push rod. The push rod can be configured to slide longitudinally between a proximal position and a distal position to transition the expandable basket assembly between an expanded configuration and a collapsed configuration. The method can include attaching a plurality of electrodes to at least the second plurality of spines.

The disclosed technology can include a medical probe, comprising a tubular shaft including a proximal end and a distal end. The tubular shaft can extend along a longitudinal axis. The medical probe can comprise an expandable basket assembly coupled to the distal end of the tubular shaft. The basket assembly can comprise a first plurality of spines coupled to the distal end of the tubular shaft and comprising a hook member and a second plurality of spines. Each spine of the second plurality of spines comprising an aperture and being rotatably coupled to a respective spine of the first plurality of spines. The basket assembly can comprise a plurality of electrodes attached to at least the second plurality of spines. The medical probe can further include a push rod connected to a distal end of the second plurality of spines. The push rod can be configured to slide longitudinally between a proximal position and a distal position to transition the expandable basket assembly between an expanded configuration and a collapsed configuration.

### BRIEF DESCRIPTION OF THE DRAWINGS

FIG. 1 is a schematic pictorial illustration of a medical system including a medical probe whose distal end has a basket assembly with electrodes coupled together, in accordance with an embodiment of the disclosed technology;
FIG. 2A is a perspective view of an expandable basket assembly in an expanded configuration, in accordance with the disclosed technology;
FIG. 2B is a detail view of a hinge of the expandable basket assembly of FIG. 2A in an assembled configuration, in accordance with the disclosed technology;
FIG. 2C is a detail view of a hinge of the expandable basket assembly in an disassembled configuration, in accordance with the disclosed technology;
FIG. 2D is a perspective view of an expandable basket assembly in a semi-collapsed configuration, in accordance with the disclosed technology;
FIG. 2E is a detail view of a hinge of the expandable basket assembly of FIG. 2D in an assembled configuration, in accordance with the disclosed technology;
FIG. 2F is a side view of the expandable basket assembly in a collapsed configuration, in accordance with the disclosed technology;
FIG. 3A is another perspective view of an expandable basket assembly in an expanded configuration, in accordance with the disclosed technology;
FIG. 3B is another perspective view of an expandable basket assembly in a semi-collapsed configuration, in accordance with the disclosed technology;
FIG. 4A is an exploded view of spines and a pin of the expandable basket assembly, in accordance with the disclosed technology;
FIG. 4B is another exploded view of spines of the expandable basket assembly with one spine having a protrusion, in accordance with the disclosed technology;
FIG. 5A is an exploded view of spines and a hinge member of the expandable basket assembly, in accordance with the disclosed technology;
FIG. 5B is another exploded view of spines and a hinge member of the expandable basket assembly, in accordance with the disclosed technology;
FIG. 6A is a perspective view of another example of the spines of the expandable basket assembly, in accordance with the disclosed technology;
FIG. 6B is a side view of another example of the spines of the expandable basket assembly, in accordance with the disclosed technology;
FIG. 6C is an exploded view of another example of the spines of the expandable basket assembly, in accordance with the disclosed technology;
FIG. 7A is another perspective view of an expandable basket assembly in an expanded configuration, in accordance with the disclosed technology;
FIG. 7B is another perspective view of an expandable basket assembly in a semi-collapsed configuration, in accordance with the disclosed technology; and
FIG. 8 is a flow chart of a method of assembling an expandable basket assembly, in accordance with the disclosed technology.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%.

As used herein, the terms "patient," "host," "user," and "subject" refer to any human or animal subject and are not intended to limit the systems or methods to human use, although use of the subject invention in a human patient represents a preferred embodiment. In addition, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example. As well, the term "proximal" indicates a location closer to the operator or physician whereas "distal" indicates a location further away to the operator or physician.

As discussed herein, "physician" or "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode including a high current density and high electric flux density is positioned at a treatment site, and a second electrode including comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal including a positive-voltage phase pulse (referred to herein as "positive phase") and a negative-voltage phase pulse (referred to herein as "negative phase"). "Monophasic pulse" refers to an electrical signal including only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape including an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, although the tubular structures may be generally illustrated as a substantially right cylindrical structure, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

Example systems, methods, and devices of the present disclosure may be particularly suited for IRE ablation of cardiac tissue to treat cardiac arrhythmias. Ablative energies are typically provided to cardiac tissue by electrodes which can deliver ablative energy alongside the tissue to be ablated. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by programmed cell death or apoptosis, which is believed to leave less scar tissue as compared to other ablation modalities. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

Manufacturing and constructing of a medical probe capable of IRE as discussed above will now be discussed. The solution of this disclosure includes systems and methods for constructing a basket assembly. By coupling a plurality of spine struts together forming a plurality of hinges, the basket assembly can be configured to transition between an expanded configuration and a collapsed configuration. Furthermore, the hinges enable the basket assembly to form a more compact shape when in a collapsed configuration. Further still, the hinges reduce the stress concentrations which can be present in spine, thereby reducing the likelihood that the spines may break when flexed.

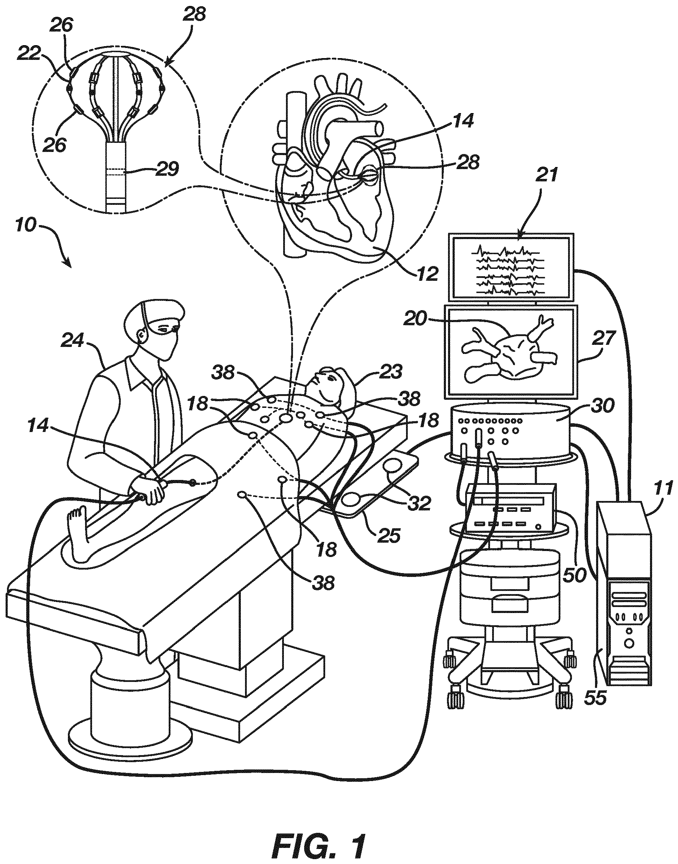

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a distal tip 28 of catheter 14 into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 26 optionally distributed over a plurality of spines 22 at distal tip 28 (also referred to herein as a "basket assembly" or "expandable basket assembly") and configured to sense the IEGM signals. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; and 6,892,091, each of which is incorporated herein by reference as if set forth fully herein and are included in the appendix attached hereto.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which is incorporated herein by reference as if set forth fully herein and are included in the appendix attached hereto.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

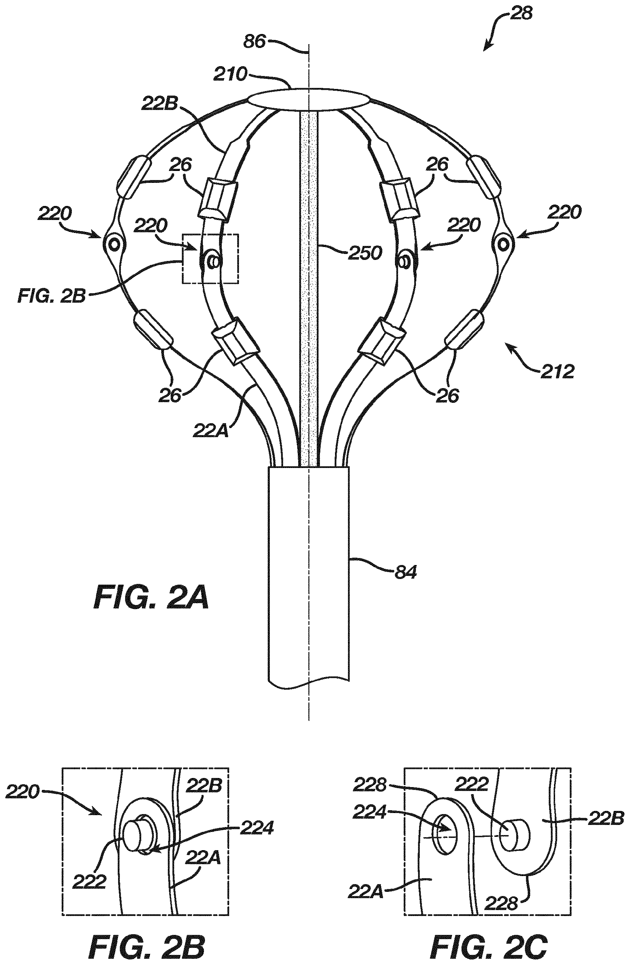

FIG. 2A is a perspective view of an expandable basket assembly 28 in an expanded configuration, in accordance with the disclosed technology. As shown in FIG. 2A, the basket assembly 28 can include a plurality of spines 22A, 22B that bow radially outward from a longitudinal axis 86 when in the expanded configuration. The plurality of spines 22A, 22B can further include a plurality of electrodes 26 disposed thereon that can be configured for mapping of electrophysiological signals and/or ablation of tissue.

The spines 22A, 22B can include a first plurality of spines 22A that can be attached to a distal end of a tubular shaft 84 and a second plurality of spines 22B that can be rotatably attached to the first plurality of spines 22A by a plurality of hinges 220. In this way, the second plurality of spines 22B can be configured to rotate in relation to the first plurality of spines 22A, thereby facilitating transition between the expanded configuration and the collapsed configuration. In other words, the first spines 22A can form a proximal portion of the basket assembly 28 and the second spines 22B can form a distal portion of the basket assembly 28 and the second spines 22B can rotate outwardly and inwardly in relation to the first spines 22A such that the distal portion of the basket assembly 28 can move in relation to the proximal portion.

The basket assembly 28 can further include a push rod 250 that can be attached to a spine intersection 210 that is disposed at a distal end of the second plurality of spines 22B. The push rod 250 can be configured to slide along the longitudinal axis 86 to transition the basket assembly 28 between the collapsed and expanded configurations. The spine intersection 210 can form an atraumatic tip to prevent damage to tissue. Furthermore, the spine intersection 210 can connect each of the second plurality of spines 22B to each other to form a unitary assembly. In some examples, the spine intersection 210 and the second plurality of spines 22B can be a single piece of continuous material such as nitinol, cobalt chromium, stainless steel, titanium, or another resilient biocompatible material such as a polymer material. Similarly, the first plurality of spines 22A can be made from nitinol, cobalt chromium, stainless steel, titanium, or another resilient biocompatible material such as a polymer material. The first plurality of spines 22A and the second plurality of spines 22B can be configured to form an approximately spherical or approximately oblate-spheroid shaped basket assembly 28.

The electrodes 26 can be any type of electrode configured including, but not limited to, electrodes suitable for sensing electrophysiological signals, ablation, position sensing, reference electrodes, etc. The electrodes 26 can be ring-type electrodes, bulging-type electrodes, rectangular electrodes, circular electrodes, etc. Furthermore, the electrodes 900 can be configured to deliver electrical pulses for irreversible electroporation, the pulses having a peak voltage of at least 900 volts (V).

The hinges 220, as shown in FIGs. 2B and 2C, can include a protrusion 222 that can extend through an aperture 224. As shown in FIGs. 2B and 2C, the protrusion 222 can extend outwardly from the second spine 22B and the aperture 224 can extend through the first spine 22A. Alternatively, the protrusion 222 can extend outwardly from the first spine 22A and the aperture 224 can extend through the second spine 22B. The protrusion 222 can be configured to form a snap-fit with the aperture 224 when assembled together, thereby securing the first spine 22A to the second spine 22B but allowing for rotation of the second spines 22B in relation to the first spines 22A. The protrusion 222 can have smooth or rounded edges and be configured to be approximately flush with an outer-facing surface of the first spine 22A to reduce sharp edges which could damage tissue. Furthermore, as shown in FIG. 2C, the first spine 22A and the second spine 22B can each have rounded ends 228 to reduce the chances of the basket assembly 28 damaging tissue and to reduce the likelihood that the hinge 220 becomes bound or otherwise is unable to rotate.

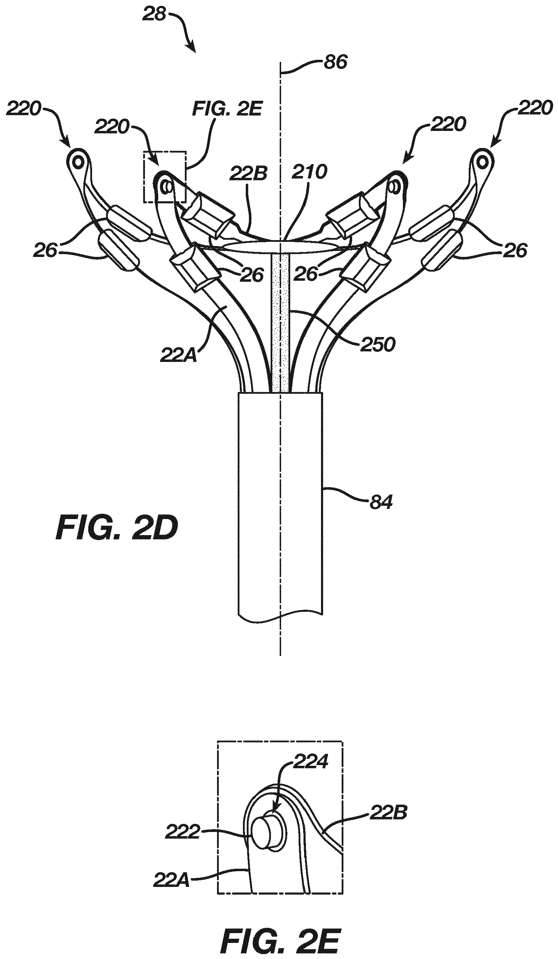

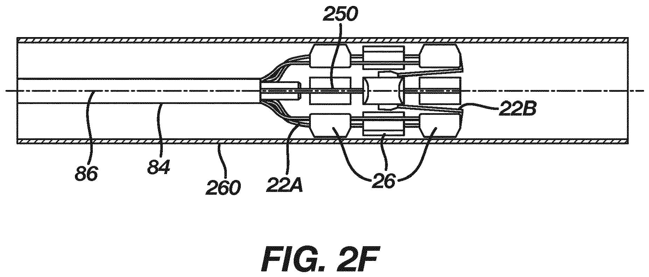

FIG. 2D is a perspective view of an expandable basket assembly 28 in a semi-collapsed configuration, in accordance with the disclosed technology. As shown in FIG. 2D and 2E, the second spines 22B can rotate and be at least partially nested in the portion of the basket assembly 28 formed by the first spines 22A. For example, when the push rod 250 is pulled proximally, the spine intersection 210 and the second spines 22B will be pulled inwardly into the basket assembly 28 and eventually nested in the first spines 22A. The basket assembly 28 can be collapsed, for example, when the physician 24 retracts the basket assembly 28 back into an insertion sheath 260 to remove the basket assembly 28 from the patient 23. FIG. 2F illustrates the basket assembly 28 retracted or otherwise position in the insertion sheath 260. As shown, the second spines 22B can be nested in or between the first spines 22A when the basket assembly 28 is in the collapsed configuration.

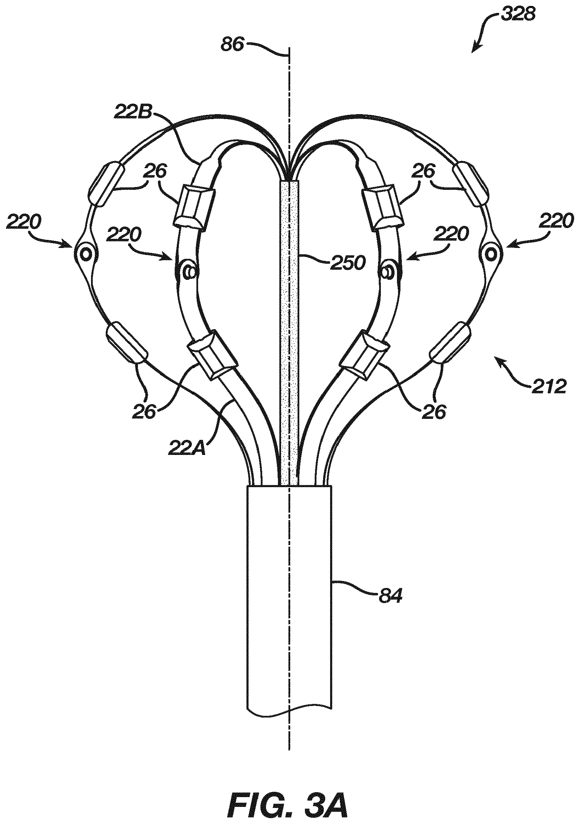

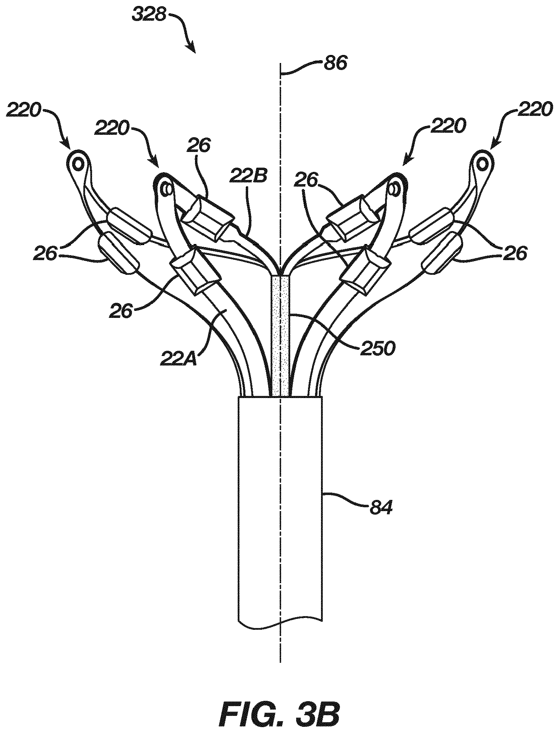

FIGs. 3A and 3B illustrate another expandable basket assembly 328 in an expanded configuration (FIG. 3A) and in a collapsed configuration (FIG. 3B), in accordance with the disclosed technology. The basket assembly 328 can include all of the same features as the basket assembly 28 except that basket assembly 328 does not include a spine intersection 210. Rather, the distal ends of the second spines 22B of the basket assembly 328 are directly attached to the push rod 250. Depending on how the second spines 22B are attached to the push rod 250, the spines 22B can be naturally biased outwardly to help expand the basket assembly 328 to the expanded state when the push rod 250 is push distally. In some examples the second spines 22B can be coated or otherwise covered in a polymer material to help create an atraumatic tip. For example, the distal ends of the second spines 22B can be dipped in a polymer material that helps to prevent the second spines 22B from damaging tissue.

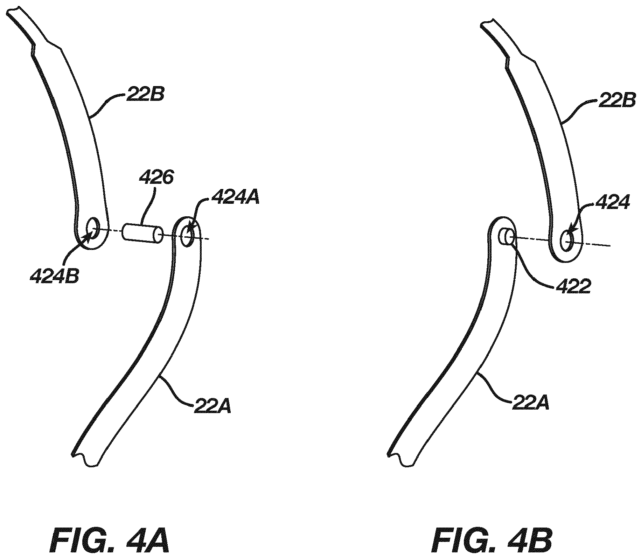

FIG. 4A is an exploded view of a first spine 22A, a second spine 22B, and a pin 426 of the expandable basket assembly 28, in accordance with the disclosed technology. As shown, the first spine 22A and the second spine 22B can be attached to each other with a pin 426 that can be inserted into a first aperture 424A on the first pine 22A and a second aperture 424B on the second spine 22B. The pin 426 can be configured to form a snap fit with the first aperture 424A and the second aperture 424B to attach the first spine 22A to the second spine 22B.

FIG. 4B is another exploded view of a first spine 22A and a second spine 22B of the expandable basket assembly 28, in accordance with the disclosed technology. As explained supra, another method of attaching a first spine 22A to a second spine 22B is to have a protrusion 422 extend through an aperture 424. The protrusion 422 can be on the first spine 22A and the aperture 424 can be on the second spine 22B. Alternatively, the protrusion 422 can be on the second spine 22B and the aperture 424 can be on the first spine 22A. The protrusion 424 can be configured to form a snap-fit with the aperture 424, thereby securing the first spine 22A to the second spine 22B but allowing for rotation of the second spine 22B in relation to the first spine 22A. In other examples, the protrusion 422 can be inserted into the aperture 424 and then the end of the protrusion 422 can be bent or otherwise enlarged to prevent the protrusion 422 from being pulled out of the aperture 424.

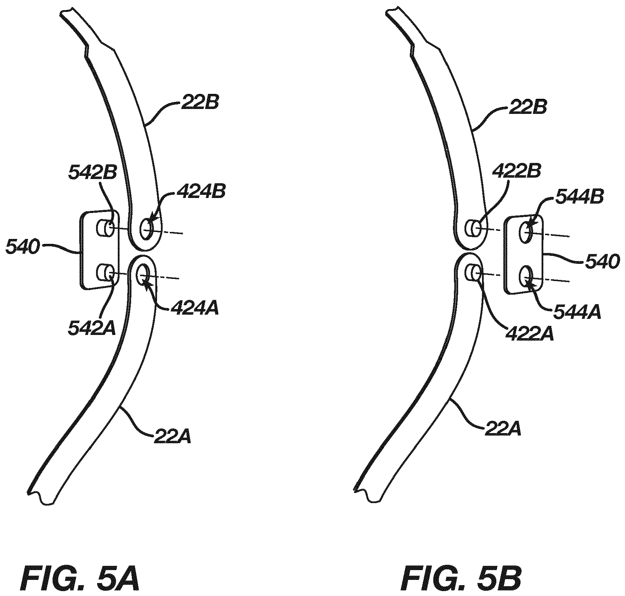

FIGs. 5A and 5B are exploded views of first spines 22A, second spines 22B, and hinge members 540, in accordance with the disclosed technology. By having a hinge member 540, the basket assembly 28 can allow for greater movement of the second spines 22B in relation to the first spines 22A. This can help to facilitate an easier transition between a collapsed configuration and an expanded configuration.

As shown in FIG. 5A, the first spine 22A and the second spine 22B can each have an aperture 424A, 424B and a hinge member 540 can be positioned between the first spine 22A and the second spine 22B. The hinge member 540 can have a first protrusion 542A positioned more proximally than a second protrusion 542B. Similar to the examples described supra, the first protrusion 542A can be configured to extend through the first aperture 424 to connect the first spines 22A to the hinge member 540 and the second protrusion 542B can be configured to extend through the second aperture 424A to connect the hinge member 540 to the second spine 22B. As before, the protrusions 542A, 542B can be configured to form a snap-fit with the aperture 424A, 424B or otherwise be prevented from being removed from the aperture 424A, 424B.

FIG. 5B shows a similar configured as FIG. 5A except that the first spine 22A and the second spine 22B each have the protrusions 422A, 422B and the hinge member 540 has apertures 544A, 544B. As before the protrusions 422A, 422B can be configured to extend at least partially through the apertures 544A, 544B to attach the first spine 22A and the second spine 22B to the hinge member 540.

Although not shown, it will be appreciated by one of skill in the art that the examples shown in FIGs. 5A and 5B could also include apertures formed in each of the first spine 22A, the second spine 22B, and the hinge member 540 and pins can extend through each of the apertures to connect the first spine 22A and the second spine 22B to the hinge member 540.

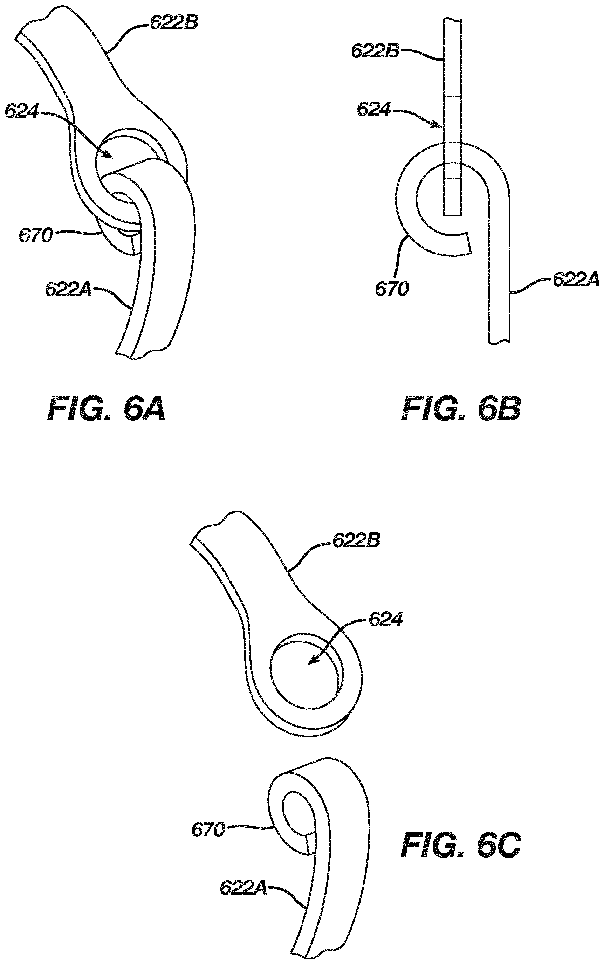

FIGs. 6A, 6B, and 6C illustrate another example of spines 622A, 622B configured as hinges. For example, the disclosed technology can include a first spine 622A having a hook member 670 that is configured to be bent through an aperture 624 of a second spine 622B. The hook member 670 can be configured to remain somewhat loose around the second spine 622 such that the first spine 622A can rotate with respect to the first spine 622B. As shown, the hook member 670 can be an end of the first spine 622A that is bent or otherwise formed into a hook shape that can be fitted through the aperture 624 of the second spine 622B to rotatably connect the first spine 622A to the second spine 622B. It will be appreciated that, in other examples, the second spine 622B can have the hook member 670 and the first spine 622A can include the aperture 624 without departing from the scope of this disclosure.

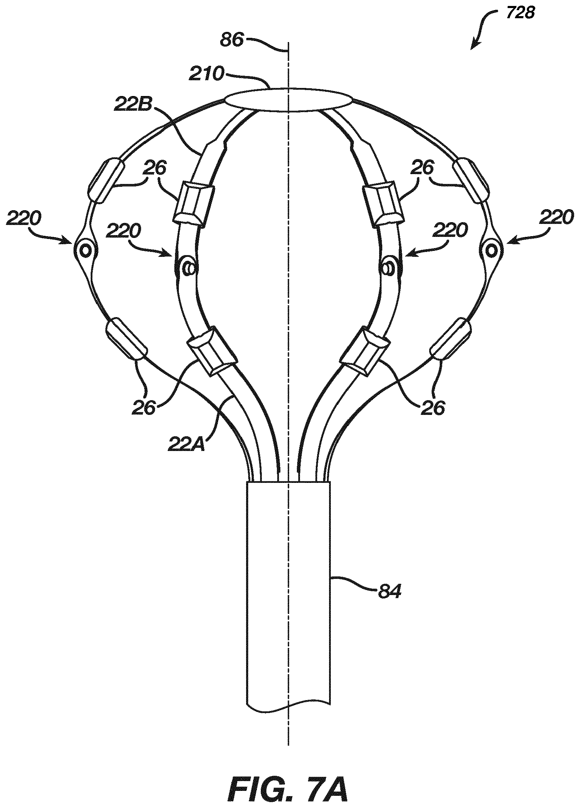

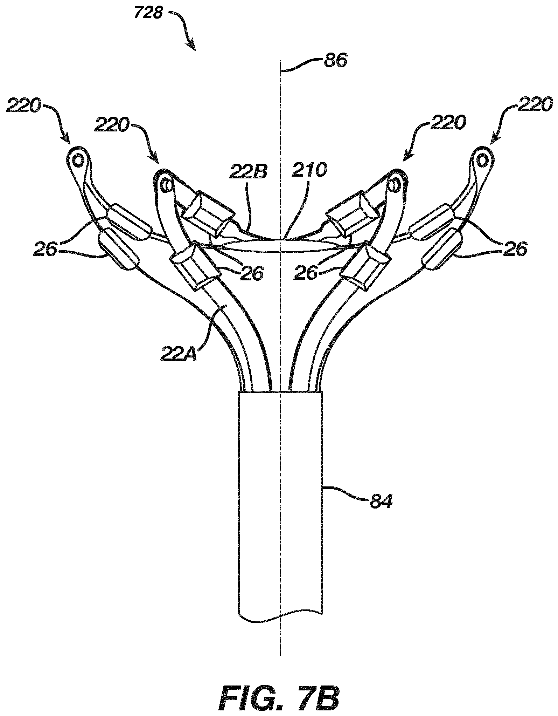

FIGs. 7A and 7B illustrate is another example basket assembly 728 in an expanded configuration (FIG. 7A) and in a semi-collapsed configuration (FIG. 7B), in accordance with the disclosed technology. The basket assembly 728 can include each of the same features described in relation to the basket assembly 28 except that the basket assembly 728 does not include a push rod 250. Rather, at least one of the first spines 22A and the second spines 22B can be formed with a bias such that the first spines 22A and the second spines 22B are naturally biased to be in the expanded configuration. Thus, when the basket assembly 728 is pushed from the delivery sheath 260, the basket assembly 728 can naturally transition to the expanded configuration. For example, the first spines 22A, the second spines 22B, or both, can be formed from nitinol and formed in such a way that the first spines 22A and/or the second spines 22B have a natural tendency to expand outward to the expanded configuration.

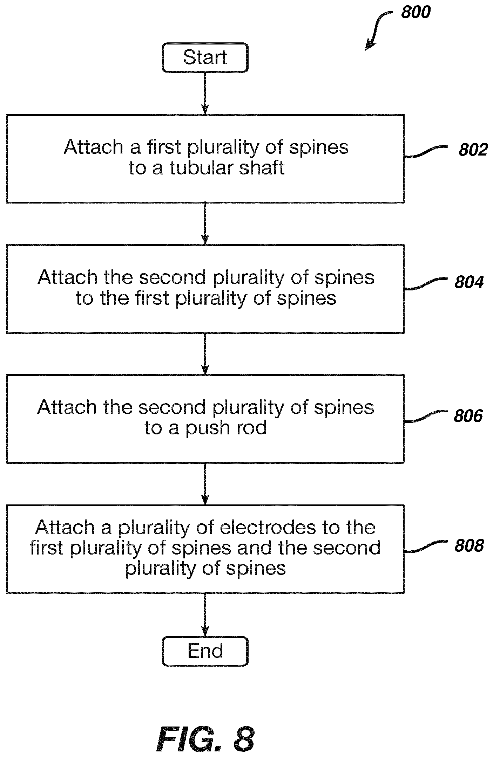

FIG. 8 is a flow chart of a method 800 of assembling an expandable basket assembly, in accordance with the disclosed technology. The basket assembly can include all of the same features as the basket assemblies 28, 328, 728 described further herein. The method 800 can include attaching 802 a first plurality of spines (e.g., first spines 22A) to a tubular shaft (e.g., tubular shaft 84. The method 800 can include attaching 804 a second plurality of spines (e.g., second spines 22B) to the first plurality of spines. Attaching 804 the second plurality of spines to the first plurality of spines can include any of the methods of attachment described herein. Furthermore, although not shown in FIG. 8, Attaching 804 the second plurality of spines to the first plurality of spines can include attaching the first plurality of spines and the second plurality of spines to a hinge member (e.g., hinge member 540) according to the examples shown and described herein. The method 800 can further include attaching 806 the second plurality of spines to a push rod (e.g., push rod 250) and attaching 808 a plurality of electrodes (e.g., electrodes 26) to the first plurality of spines and the second plurality of spines.

As will be appreciated by one skilled in the art, method 800 can include any of the various features of the disclosed technology described herein and can be varied depending on the particular configuration. Thus, method 800 should not be construed as limited to the particular steps and order of steps explicitly described herein. Furthermore, the order of steps provided herein are offered as an example and the steps can be performed in various other orders or include other steps in between those set forth above.

The disclosed technology described herein can be further understood according to the following clauses:
Clause 1: A medical probe, comprising: a tubular shaft including a proximal end and a distal end, the tubular shaft extending along a longitudinal axis; and an expandable basket assembly coupled to the distal end of the tubular shaft, the basket assembly comprising: a first plurality of spines coupled to the distal end of the tubular shaft; a second plurality of spines, each spine of the second plurality of spines rotatably coupled to a respective spine of the first plurality of spines; and a plurality of electrodes attached to at least the second plurality of spines; and a push rod connected to a distal end of the second plurality of spines, the push rod configured to slide longitudinally between a proximal position and a distal position to transition the expandable basket assembly between an expanded configuration and a collapsed configuration.
Clause 2: The medical probe of clause 1, the first plurality of spines forming a proximal portion of the expandable basket assembly and the second plurality of spines forming a distal portion of the expandable basket assembly.
Clause 3: The medical probe of clause 2, the second plurality of spines configured to rotate outwardly with respect to the first plurality of spines to transition to an expanded configuration.
Clause 4: The medical probe of any of clauses 2 or 3, the second plurality of spines configured to rotate inwardly with respect to the first plurality of spines such that distal portion of the expandable basket assembly nests at least partially within the proximal portion of the expandable basket assembly.
Clause 5: The medical probe of any of the preceding clauses, the second plurality of spines being attached to each other to form a unitary spine assembly.
Clause 6: The medical probe of clause 5, the second plurality of spines comprising a single piece of continuous biocompatible material.
Clause 7: The medical probe of any of the preceding clauses, each spine of the first plurality of spines comprising a first aperture extending therethrough at its distal end, each spine of the second plurality of spines comprising a second aperture extending therethrough at its proximal end, and the medical probe further comprising a plurality of pins, each pin of the plurality of pins configured to extend through a respective first aperture and a respective second aperture to rotatably couple the first plurality of spines to the second plurality of spines.
Clause 8: The medical probe of clause 4, the pin forming a snap-fit with the first plurality of spines and the second plurality of spines to secure the first plurality of spines to the second plurality of spines.
Clause 9: The medical probe according to any of clauses 1-6, each spine of the first plurality of spines comprising a protrusion, each spine of the second plurality of spines comprising an aperture extending therethrough, the aperture configured to receive the protrusion to rotatably couple the first plurality of spines to the second plurality of spines.
Clause 10: The medical probe of clause 9, the protrusion forming a snap-fit with the aperture to secure the first plurality of spines to the second plurality of spines.
Clause 11: The medical probe according to any of clauses 1-6, each spine of the second plurality of spines comprising a protrusion, each spine of the first plurality of spines comprising an aperture extending therethrough, the aperture configured to receive the protrusion to rotatably couple the first plurality of spines to the second plurality of spines.
Clause 12: The medical probe of clause 11, the protrusion forming a snap-fit with the aperture to secure the first plurality of spines to the second plurality of spines.
Clause 13: The medical probe according to any of clauses 1-6, further comprising a plurality of hinge members, each hinge member of the plurality of hinge members being coupled to a distal end of a respective spine of the first plurality of spines and to a proximal end of a respective spine of the second plurality of spines.
Clause 14: The medical probe of clause 13, each hinge member comprising a proximal aperture and a distal aperture, the proximal aperture configured to receive a protrusion of a respective spine of the first plurality of spines and the distal aperture configured to receive a protrusion of a respective spine of the second plurality of spines to rotatably attach the first plurality of spines, the second plurality of spines, and the plurality of hinge members to each other.
Clause 15: The medical probe of clause 13, each hinge member comprising a proximal protrusion and a distal protrusion, the proximal protrusion configured to be inserted into an aperture of a respective spine of the first plurality of spines and the distal protrusion configured to be inserted into and aperture of a respective spine of the second plurality of spines to rotatably attach the first plurality of spines, the second plurality of spines, and the plurality of hinge members to each other.
Clause 16: The medical probe according to any of the preceding clauses, wherein each spine of the first plurality of spines and the second plurality of spines comprises a material selected from a group consisting of nitinol, cobalt chromium, stainless steel, titanium.
Clause 17: The medical probe according to any of the preceding clauses, wherein each spine of the first plurality of spines and the second plurality of spines comprises a polymer.
Clause 18: The medical probe according to any of the preceding clauses, wherein each electrode of the plurality of electrodes comprises a ring type electrode.
Clause 19: The medical probe according to any of clauses 1-18, wherein each electrode of the plurality of electrodes comprises a bulging type electrode.
Clause 20: The medical probe according to any of clauses 1-18, wherein each electrode of the plurality of electrodes comprises a rectangular electrode.
Clause 21: The medical probe according to any of the preceding clauses, wherein the plurality of electrodes is configured to deliver electrical pulses for irreversible electroporation, the pulses having a peak voltage of at least 900 volts (V).
Clause 22: The medical probe according to any of the preceding clauses, wherein the expandable basket assembly is configured to form an approximately spherically-shaped basket assembly when in the expanded form.
Clause 23: The medical probe according to any of clauses 1-22, wherein the expandable basket assembly is configured to form an approximately oblate-spheroid basket assembly when in the expanded form.
Clause 24: A medical probe, comprising: a tubular shaft including a proximal end and a distal end, the tubular shaft extending along a longitudinal axis; and an expandable basket assembly coupled to the distal end of the tubular shaft, the basket assembly comprising: a first plurality of spines coupled to the distal end of the tubular shaft; a second plurality of spines, each spine of the second plurality of spines rotatably coupled to a respective spine of the first plurality of spines, the second plurality of spines being biased to bow distally with respect to the first plurality of spines to cause the expandable basket assembly to transition to an expanded configuration.
Clause 25: A method of constructing a medical probe, the method comprising: attaching a first plurality of spines to a tubular shaft, the tubular shaft extending along a longitudinal axis; attaching a second plurality of spines to a distal end of the first plurality of spines, the second plurality of spines being rotatably attachable to the first plurality of spines, the first plurality of spines and the second plurality of spines forming an expandable basket assembly; attaching a distal end of the second plurality of spines to a push rod, the push rod configured to slide longitudinally between a proximal position and a distal position to transition the expandable basket assembly between an expanded configuration and a collapsed configuration; and attaching a plurality of electrodes to at least the second plurality of spines.
Clause 26: The method of clause 25, the first plurality of spines forming a proximal portion of the expandable basket assembly and the second plurality of spines forming a distal portion of the expandable basket assembly.
Clause 27: The method of clause 26, the second plurality of spines configured to rotate outwardly with respect to the first plurality of spines to transition to an expanded configuration.
Clause 28: The method of any of clauses 26 or 27, the second plurality of spines configured to rotate inwardly with respect to the first plurality of spines such that distal portion of the expandable basket assembly nests at least partially within the proximal portion of the expandable basket assembly.
Clause 29: The method of any of clauses 25-27, the second plurality of spines being attached to each other to form a unitary spine assembly.
Clause 30: The method of clause 29, the second plurality of spines comprising a single piece of continuous biocompatible material.
Clause 31: The medical of any of clauses 25-30, each spine of the first plurality of spines comprising a first aperture extending therethrough at its distal end, each spine of the second plurality of spines comprising a second aperture extending therethrough at its proximal end, and the method further comprising: aligning the first aperture with the second aperture and insert a pin into the first aperture and the second aperture, the plurality of pins configured to extend through a respective first aperture and a respective second aperture to rotatably couple the first plurality of spines to the second plurality of spines.
Clause 32: The method of clause 31, the pin forming a snap-fit with the first plurality of spines and the second plurality of spines to secure the first plurality of spines to the second plurality of spines.
Clause 33: The method according to any of clauses 25-30, each spine of the first plurality of spines comprising a protrusion, each spine of the second plurality of spines comprising an aperture extending therethrough, the method further comprising inserting the protrusion into the aperture to rotatably couple the first plurality of spines to the second plurality of spines.
Clause 34: The method of clause 33, the protrusion forming a snap-fit with the aperture to secure the first plurality of spines to the second plurality of spines.
Clause 35: The method according to any of clauses 25-30, each spine of the second plurality of spines comprising a protrusion, each spine of the first plurality of spines comprising an aperture extending therethrough, the method further comprising inserting the protrusion into the aperture to rotatably couple the first plurality of spines to the second plurality of spines.
Clause 36: The method of clause 35, the protrusion forming a snap-fit with the aperture to secure the first plurality of spines to the second plurality of spines.
Clause 37: The method according to any clauses 25-30, further comprising: attaching each spine of the first plurality of spines to a hinge member of a plurality of hinge members; and attaching each spine of the second plurality of spines to the hinge member of the plurality of hinge members, to rotatably attach the first plurality of spines, the second plurality of spines, and the plurality of hinge members to each other.
Clause 38: The method of clause 37, each hinge member comprising a proximal aperture and a distal aperture, the proximal aperture configured to receive a protrusion of a respective spine of the first plurality of spines and the distal aperture configured to receive a protrusion of a respective spine of the second plurality of spines.
Clause 39: The method of clause 37, each hinge member comprising a proximal protrusion and a distal protrusion, the proximal protrusion configured to be inserted into an aperture of a respective spine of the first plurality of spines and the distal protrusion configured to be inserted into and aperture of a respective spine of the second plurality of spines.
Clause 40: The method according to any of clauses 25-39, wherein each spine of the first plurality of spines and the second plurality of spines comprises a material selected from a group consisting of nitinol, cobalt chromium, stainless steel, titanium.
Clause 41: The method according to any of clauses 25-40, wherein each spine of the first plurality of spines and the second plurality of spines comprises a polymer.
Clause 42: The method according to any of clauses 25-41, wherein each electrode of the plurality of electrodes comprises a ring type electrode.
Clause 43: The method according to any of clauses 25-41, wherein each electrode of the plurality of electrodes comprises a bulging type electrode.
Clause 44: The method according to any of clauses 25-41, wherein each electrode of the plurality of electrodes comprises a rectangular electrode.
Clause 45: The method according to any of clauses 25-44, wherein the plurality of electrodes is configured to deliver electrical pulses for irreversible electroporation, the pulses having a peak voltage of at least 900 volts (V).
Clause 46: The method according to any of clauses 25-45, wherein the expandable basket assembly is configured to form an approximately spherically-shaped basket assembly when in the expanded form.
Clause 47: The method according to any of clauses 25-45, wherein the expandable basket assembly is configured to form an approximately oblate-spheroid basket assembly when in the expanded form.
Clause 48: A medical probe, comprising: a tubular shaft including a proximal end and a distal end, the tubular shaft extending along a longitudinal axis; and an expandable basket assembly coupled to the distal end of the tubular shaft, the basket assembly comprising: a first plurality of spines coupled to the distal end of the tubular shaft and comprising a hook member; a second plurality of spines, each spine of the second plurality of spines comprising an aperture and being rotatably coupled to a respective spine of the first plurality of spines; and a plurality of electrodes attached to at least the second plurality of spines; and a push rod connected to a distal end of the second plurality of spines, the push rod configured to slide longitudinally between a proximal position and a distal position to transition the expandable basket assembly between an expanded configuration and a collapsed configuration.

The embodiments described above are cited by way of example, and the present invention is not limited by what has been particularly shown and described hereinabove. Rather, the scope of the invention includes both combinations and sub combinations of the various features described and illustrated hereinabove, as well as variations and modifications thereof which would occur to persons skilled in the art upon reading the foregoing description and which are not disclosed in the prior art.

## Claims

1. A medical probe, comprising:
a tubular shaft including a proximal end and a distal end, the tubular shaft extending along a longitudinal axis; and
an expandable basket assembly coupled to the distal end of the tubular shaft, the basket assembly comprising:
a first plurality of spines coupled to the distal end of the tubular shaft;
a second plurality of spines, each spine of the second plurality of spines rotatably coupled to a respective spine of the first plurality of spines; and
a plurality of electrodes attached to at least the second plurality of spines; and
a push rod connected to a distal end of the second plurality of spines, the push rod configured to slide longitudinally between a proximal position and a distal position to transition the expandable basket assembly between an expanded configuration and a collapsed configuration.

2. The medical probe of claim 1, the first plurality of spines forming a proximal portion of the expandable basket assembly and the second plurality of spines forming a distal portion of the expandable basket assembly.

3. The medical probe of claim 2, the second plurality of spines configured to rotate outwardly with respect to the first plurality of spines to transition to an expanded configuration.

4. The medical probe of claim 2 or claim 3, the second plurality of spines configured to rotate inwardly with respect to the first plurality of spines such that distal portion of the expandable basket assembly nests at least partially within the proximal portion of the expandable basket assembly.

5. The medical probe of any preceding claim, the second plurality of spines being attached to each other to form a unitary spine assembly, optionally the second plurality of spines comprising a single piece of continuous biocompatible material.

6. The medical probe of any preceding claim, each spine of the first plurality of spines comprising a first aperture extending therethrough at its distal end,
each spine of the second plurality of spines comprising a second aperture extending therethrough at its proximal end, and
the medical probe further comprising a plurality of pins, each pin of the plurality of pins configured to extend through a respective first aperture and a respective second aperture to rotatably couple the first plurality of spines to the second plurality of spines,
optionally the pin forming a snap-fit with the first plurality of spines and the second plurality of spines to secure the first plurality of spines to the second plurality of spines.

7. The medical probe of any preceding claim, each spine of the first plurality of spines comprising a protrusion,
each spine of the second plurality of spines comprising an aperture extending therethrough, the aperture configured to receive the protrusion to rotatably couple the first plurality of spines to the second plurality of spines,
optionally the protrusion forming a snap-fit with the aperture to secure the first plurality of spines to the second plurality of spines.

8. The medical probe of any of claims 1 to 5, each spine of the second plurality of spines comprising a protrusion,
each spine of the first plurality of spines comprising an aperture extending therethrough, the aperture configured to receive the protrusion to rotatably couple the first plurality of spines to the second plurality of spines,
optionally the protrusion forming a snap-fit with the aperture to secure the first plurality of spines to the second plurality of spines.

9. The medical probe of any of claims 1 to 5, further comprising a plurality of hinge members, each hinge member of the plurality of hinge members being coupled to a distal end of a respective spine of the first plurality of spines and to a proximal end of a respective spine of the second plurality of spines.

10. The medical probe of claim 9, each hinge member comprising i) a proximal aperture and a distal aperture, the proximal aperture configured to receive a protrusion of a respective spine of the first plurality of spines and the distal aperture configured to receive a protrusion of a respective spine of the second plurality of spines to rotatably attach the first plurality of spines, the second plurality of spines, and the plurality of hinge members to each other, or ii) a proximal protrusion and a distal protrusion, the proximal protrusion configured to be inserted into an aperture of a respective spine of the first plurality of spines and the distal protrusion configured to be inserted into and aperture of a respective spine of the second plurality of spines to rotatably attach the first plurality of spines, the second plurality of spines, and the plurality of hinge members to each other.

11. The medical probe of any preceding claim, wherein the plurality of electrodes is configured to deliver electrical pulses for irreversible electroporation, the pulses having a peak voltage of at least 900 volts (V).

12. The medical probe of any preceding claim, wherein the expandable basket assembly is configured to form an approximately spherically-shaped basket assembly when in the expanded form.

13. The medical probe of any preceding claim, wherein the expandable basket assembly is configured to form an approximately oblate-spheroid basket assembly when in the expanded form.

14. A medical probe, comprising:
a tubular shaft including a proximal end and a distal end, the tubular shaft extending along a longitudinal axis; and
an expandable basket assembly coupled to the distal end of the tubular shaft, the basket assembly comprising:
a first plurality of spines coupled to the distal end of the tubular shaft;
a second plurality of spines, each spine of the second plurality of spines rotatably coupled to a respective spine of the first plurality of spines, the second plurality of spines being biased to bow distally with respect to the first plurality of spines to cause the expandable basket assembly to transition to an expanded configuration.

15. A medical probe, comprising:
a tubular shaft including a proximal end and a distal end, the tubular shaft extending along a longitudinal axis; and
an expandable basket assembly coupled to the distal end of the tubular shaft, the basket assembly comprising:
a first plurality of spines coupled to the distal end of the tubular shaft and comprising a hook member;
a second plurality of spines, each spine of the second plurality of spines comprising an aperture and being rotatably coupled to a respective spine of the first plurality of spines; and
a plurality of electrodes attached to at least the second plurality of spines; and
a push rod connected to a distal end of the second plurality of spines, the push rod configured to slide longitudinally between a proximal position and a distal position to transition the expandable basket assembly between an expanded configuration and a collapsed configuration.
